# EUROPEAN PATENT SPECIFICATION

(11) **EP 2 787 940 B1**
(45) Date of publication and mention of the grant of the patent: **24.01.2018**
(21) Application number: 12809310.1
(22) Date of filing: 10.12.2012
(51) Int. Cl.: A61F 6/00, A61F 6/04

(54) **A PACK CONTAINING A CONDOM**
KONDOMPACKUNG
EMBALLAGE CONTENANT UN PRÉSERVATIF

(30) Priority: 09.12.2011 GB 201121204
(43) Date of publication of application: 15.10.2014
(73) Proprietor: LRC Products Limited, Slough, Berkshire SL1 3UH (GB)
(72) Inventor: CHOPDAT, Mohammed, Hull, Yorkshire HU8 7DS (GB); HAYTO, Ian, Coventry, West Midlands CV4 8HS (GB); MAYES, Geoff, Coventry, West Midlands CV4 8HS (GB)
(74) Representative: Cawdell, Karen Teresa
(86) International application number: PCT/GB2012/053069
(87) International publication number: WO 2013/084007

(56) References cited:
- WO-A1-92/20595
- WO-A1-95/02379
- WO-A1-96/29262
- WO-A2-2008/061522
- DE-A1-102007 039 837
- US-A- 4 428 747
- US-A- 5 175 142

## Description

The present invention relates to a pack containing a condom.

It is known in the art to incorporate an erectogenic or vasoactive compound into a condom. In doing this, care has to be taken as some of these compounds may have undesirable side effects for the female. As a result of this, a number of attempts have been made to keep the compound on the interior surface of the condom during storage and transportation.

US 4,829,991 discloses a compound which is sprayed, brushed or applied using a coated mandrel onto the interior surface of the condom. In an attempt to prevent migration of this layer to the outside surface of the condom, the layer has a coating and relies on friction between the penis and the condom to break the surface tension of the coating. It also suggests that, rather than rolling up the condom for packaging as is conventional, it is folded up "in accordion fashion" which seems highly impractical.

US 5,333,621 discloses the possibility of a patch which has adhesive on both sides, initially it will adhere to the condom wall, but upon the application of outside pressure, will subsequently adhere to the penis. This is difficult to manufacture and awkward to use.

US 6,080,100 discloses coatings and patches as above, and also discloses the possibility of a compartment at the tip of the condom containing the compound and separated from the remainder of the compound by a breakable internal membrane. The idea of making such a breakable membrane in a very thin flexible latex-like material and filling the resulting cavity with a compound is hard to accomplish in practice.

WO 02/078580 discloses the possibility of immobilising the compound, for example, using a thickening agent. This is similar to the coatings referred to above.

In the above prior art documents, one approach taken is to modify the condom itself, which creates complexity in manufacture and is awkward and uncomfortable for a user. Alternatively, the prior art modifies the physical properties of the compound itself. However, even then, it is difficult to prevent undesirable migration during storage. Also, the delivery may be compromised as the manner in which the compound is modified to stay in place will compromise its ability to be delivered in the most effective manner. In either case, the possibility exists for the compound to reach the 'wrong' side of the condom. This will shorten the shelf life of the product to a potentially unacceptable level.

One attempt to address this problem is disclosed in WO 95/02379. This discloses a condom pack containing a sachet which is filled with a lubricant and/or spermicide. This sachet is arranged to be opened as the condom pack is opened. However, this leads to design compromises in creating a mechanism which can simultaneously open the pack and sachet. For example, the first example discloses a tray with a lid which is pressed into the tray in order to open it. In order for this to work, the seal between the tray and the lid must be very weak and therefore prone to accidental detachment. Further, the mechanisms are potentially messy as, if the mechanisms are not operated correctly, there is the potential for the compound to be squeezed out of the pack as it is being opened, rather than being dispensed onto the condom.

A similar arrangement is disclosed in DE 10 2007 03 837, which is regarded as the closest prior art. This also discloses other examples of packs, for example, one having a compartment connected to the pack via a screw thread which is screwed into place to release the compound. Another example has a two-part tray, one containing the condom and the other containing the compound. Both trays are opened and the pack is folded together to place the compound into contact with the condom.

This document also discloses an example in which the compound is a membrane held in a bulge in the outer packaging and which has a rupturable membrane separating the compound from the condom. Pressure on the outer wall of the bulge ruptures the membrane and causes the compound to be dispensed onto the condom. The pack also requires a rigid ring between the two layers thereby introducing additional complexity into the design. As the compound is retained in a bulge in the pack, it is easy to release the compound inadvertently, particularly if the condom is being carried in a wallet or pocket where the likelihood of it being inadvertently compressed is reasonably high. In accordance with the present invention, there is provided a pack as defined in independent claim 1. The pack has an outer sealed wall containing a condom and a liquid or a gel which is isolated from the condom by a barrier, the barrier being breachable by external manipulation of the pack to apply the liquid or gel to one side of the condom without disturbing the integrity of the outer sealed wall; wherein the outer sealed wall is provided by a moulded tray sealed with a foil lid, wherein a deformable portion is provided by a centre portion of the tray, the deformable portion being deformable from a first non-deformed configuration to a second configuration in which it has expelled the liquid or gel through the barrier.

By allowing the barrier to be breached without disturbing the integrity of the outer seal wall, the mechanism for breaching the barrier and the opening mechanism can be kept separate from one another allowing each to be optimised for its own function. Further, as the liquid or gel is dispensed while the barrier is still sealed, any problems which occur with the dispensing will not squirt the liquid or gel out of the pack.

By providing the deformable portion in a moulded tray, rather than a foil, the present invention is more robust than the prior art against accidental dispensing of the liquid or gel.

This is further enhanced by an annular trough surrounding the central deformable portion. This provides a further degree of protection and also provides a convenient location for the rolled wall of the condom. The deformable portion does not project downwardly further than the lower surface of the trough. This means that, if pressed down onto a flat surface, the deformable portion is protected to some extent by the annular trough. Preferred embodiments are defined in the dependent claims.

The deformable portion is preferably arranged such that, once it has deformed more than a certain amount, it is biased to the second configuration. This has a number of advantages. The deformable portion will essentially "snap" to the second configuration caused by the biasing force. This positive movement provides a distinct feel to the user indicating to them that a deformable portion has been fully depressed. It also serves to reliably dispense as much as possible of the liquid or gel from the barrier. This contrasts with a foil where the user is solely responsible for squeezing out the liquid or gel without any assistance from the packaging.

Preferably, the pack of the deformable portion has a projection to facilitate breaching of the barrier as it is deformed.

The liquid or gel is preferably a compound including glyceryl trinitrate, or is a component including a lubricant, spermicide and/or fragrance.

Examples of packs constructed in accordance with the present invention will now be described with reference to the accompanying drawings, in which:
Fig. 1A is a perspective from above of a pack according to a first and second aspect of the invention;
Fig. 1B is a perspective from below of the first pack;
Fig. 1C is a cross-section through the first pack prior to use;
Fig. 1D is a view similar to Fig. 1C once the barrier has been breached;
Fig. 1E is a view similar to Fig. 1B once the barrier has been breached;
Fig. 1F is a view similar to Figs. 1C and 1D once the lid has been peeled off;
Fig. 1G is a view similar to Fig. 1A showing the lid peeled off;
Fig. 1H is a view similar to Fig. 1G showing removal of the condom.
The following drawings relate to other exemplary packs which are not in accordance with the present invention:
Fig. 2A is a perspective view of a first exemplary pack;
Fig. 2B is a cross-section through Fig. 2A;
Fig. 2C is a view similar to Fig. 2A showing an initial opening step;
Fig. 2D is a view similar to Fig. 2C at a later stage of opening;
Fig. 2E is a cross-section similar to Fig. 2B with the pack in the same configuration as Fig. 2D;
Fig. 2F is a perspective view showing the application of the liquid or gel to the condom;
Fig. 2G is a cross-section through the pack in the same configuration as Fig. 2F;
Fig. 2H is a perspective view showing initial opening of the pack;
Fig. 2I is a cross-sectional view showing the pack partially open;
Fig. 2J is a perspective view showing the pack fully opened;
Fig. 2K is a perspective view showing removal of the condom;
Fig. 3A is a schematic cross-sectional representation of another exemplary pack; and
Fig. 3B is a plan view of the membrane of Fig. 3A.

The present invention is for a pack containing a condom. This could be a female condom. The condom can be made of any suitable material such as natural rubber latex, polyurethane, etc.

The pack contains a liquid or gel. This is primarily intended to be an erectogenic or vasoactive compound. However, it can alternatively or additionally be a lubricant, spermicide fragrance or other type of formulation The preferred erectogenic compound is a compound containing glyceryl trinitrate (GTN). This is disclosed in WO 99/38506, to which reference is made for further details of the compound.

The pack is primarily intended to dispense a compound onto the internal surface of the condom. However, it is equally possible, simply by inverting the condom in the pack, to dispense a compound, such as a vasodilator (for example as disclosed in WO 03/088880) or Durex (RTM) Play O (RTM) gel on the opposite side. It is even within the scope of the present invention to dispense a first compound onto one side and a second compound onto the opposite side.

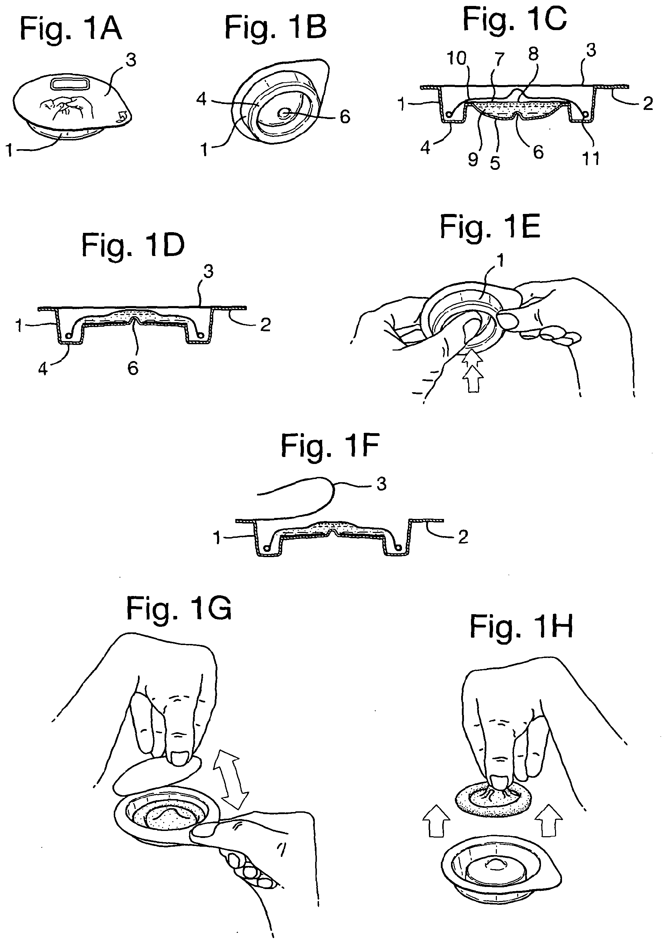

A pack in accordance with the present invention is shown in Figs. 1A-1H. This pack is based on the Durex (RTM) Deluxe pack. Essentially, it consists of a moulded tray 1 having an upper lip 2 to which a foil 3 is sealed. The tray 1 has a circular shape with an annular trough 4 around its outer periphery. This is different from the Durex (RTM) Deluxe pack which has a planar base. Inside the annular trough 4 is a depression 5 with an optional upward protrusion 6 at its centre. Sealed across the top of the depression 5 is a barrier 7 having a weakened portion 8 at its central region. The barrier may be a foil or other thin and easily tearable laminate. The weakened portion may be provided by localised thinning of the material in an area or a score line formed, for example, by laser ablation. The chamber defined by the depression 5 and barrier 7 is filled with a liquid or gel compound 9. A condom 10 is in the tray 1 on the opposite side of the barrier 7 with its outer surface uppermost. The rolled wall portion 11 of the condom 10 is accommodated within the trough 4. As can be seen from Fig. 1C, the lower wall of the trough 4 is beneath the lower wall of the depression 5 to provide some protection against accidental dispensing of the compound during transportation and storage.

In order to use the pack, the user presses on the depression 5 forcing the projection 6 through the weakened portion 8 of the membrane 7 (Figs. 1D and 1E). Once the projection reaches a certain point, the resilience of the material is such that it then tends to bias the depression to the fully depressed position. The user feels this effect as a 'snap' and may even hear an audible click. This reliably expels as much of the liquid or gel as possible, and gives the user a positive indication that they have correctly completed the dispensing operation. It also gives a clear visual indication that dispensing has been carried out. Alternatively, an increase in pressure alone can be used to penetrate the membrane. The movement of the depression 5 also forces the compound onto the lower surface of the condom. The protrusion 6 assists in transferring the maximum possible amount of the compound to the condom. The lid is then peeled off (Figs. 1F and 1G) and the condom, with the compound on its internal surface is then removed and used in the usual way. Unlike a conventional condom wrapper, the tray can accommodate the condom after use for ease of disposal.

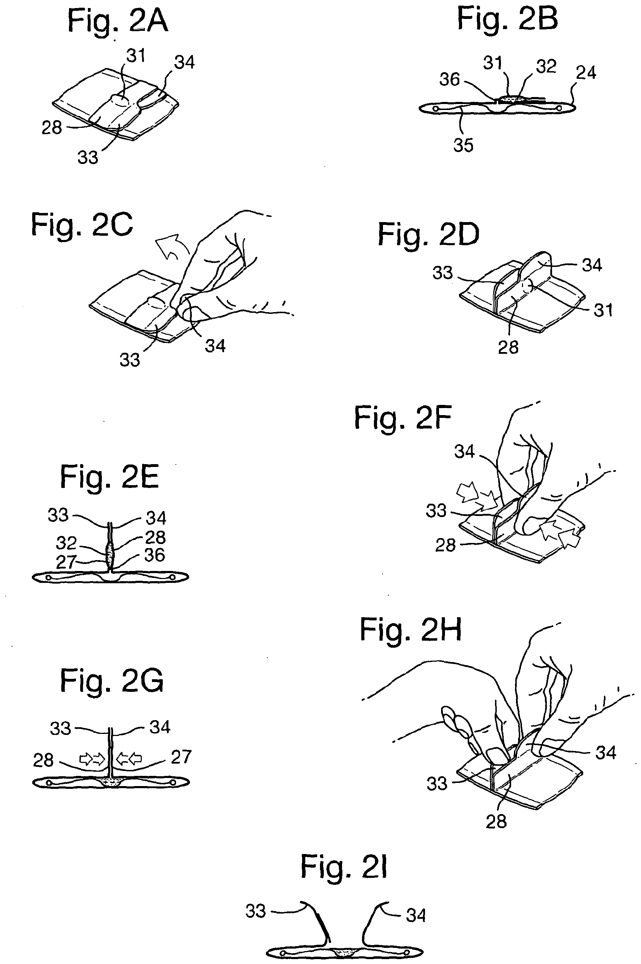

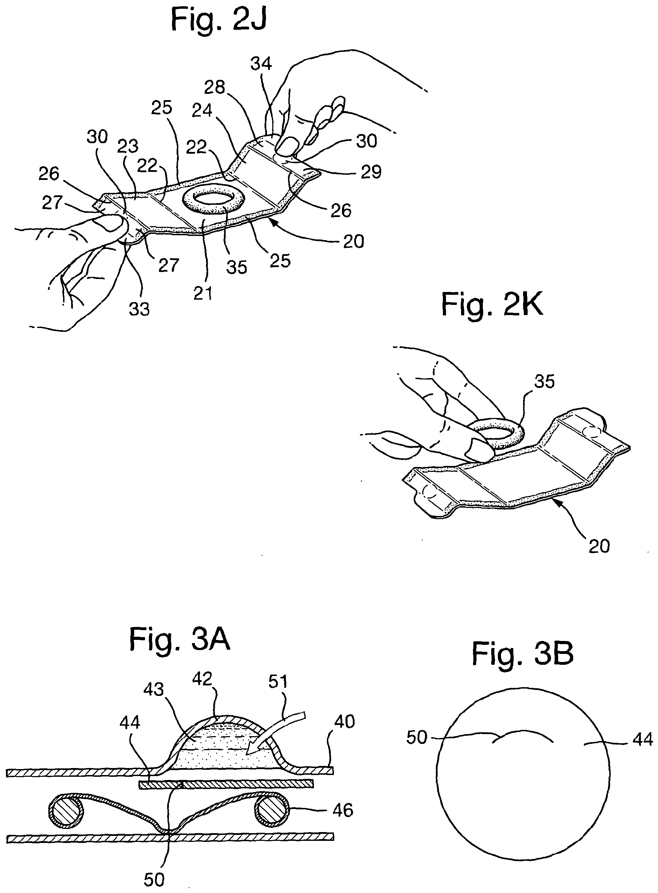

An alternative exemplary pack not in accordance with the present invention is shown in Figs. 2A to 2K. This pack is shown as being formed from a single sheet as best shown in Fig. 2J. It could also be formed from three separate sheets that are sealed together where the fold lines are shown in the figures. The sheet 20 comprises a lower wall 21 connected at either side via a fold line 22 to a respective upper wall portion 23, 24. Two strips of peelable adhesive 25 are provided along the longitudinal edges of the lower wall 21 and upper wall portions 23, 24, by virtue of which the lower wall 21 is sealed to the upper wall portions 23, 24. Alternatively, this could be a peelable weld.

At the ends opposite the fold lines 22, the upper wall portions 23, 24 are connected via second fold lines 26 with a first seam portion 27 and a second seam portion 28 respectively. The seam portions 27, 28 are coated with a peelable adhesive 29 which may be the same as the adhesive 25 or are peelable welds. However, the central region 30 of each seam portion 27, 28 is devoid adhesive to provide a compound chamber 31 which is sealed on all sides and is, in use, filled with the liquid or gel compound 32. At the end opposite to the second fold lines 26, each seam portion 27, 28 is provided with a tab 33, 34 for a user to get hold of to open the packaging as described below.

The lower wall 20 and the second side wall portions 23, 24 define a chamber containing a condom 35. The compound chamber 31 is isolated from the condom 35 by a frangible seal 36.

When initially provided to a user, the seam portions 27, 28 are folded down so that they lie flat against the underlying upper wall portion 24 as shown in Fig. 2B. This helps to preserve the integrity of the seal during transportation and storage.

In order to use the pack, the user first lifts up seam portions 27, 28 by gripping the tabs 33, 34 (see Fig. 2C) lifting the seam portions 27, 28 to an upright position (Figs. 2D and 2E). This alone may be sufficient to break the seal 36, or it may be necessary to rock the seam portion to and fro a number of times in order to do this. The user then squeezes the compound chamber 31 (Figs. 2F and 2G) to force the compound through the now opened seal 36 and on to the internal surface of the condom 35. Alternatively, the seal 36 may be weakened by being moved to the upright position and fully opened by fluid pressure as the chamber 32 is squeezed, or it may be capable of being opened by squeezing the chamber 31 with the seam portion still lying flat.

Having done this, the user then opens the pack by grasping the tabs 33, 34 (Fig. 2H) and peeling the pack open (Figs. 2I and 2J) before removing the condom, which has now been filled with the compound, and using it in the usual way. The unfolded wrapper also has the advantage it is more readily able to re-wrap the condom after use for disposal.

A further exemplary pack not in accordance with the present invention is shown in Figs. 3A and 3B.

This is based on a traditional two layer foil condom wrapper. A first layer 40 and a second layer 41 are heat sealed together around their peripheral edge in a conventional manner (the edges are not shown sealed in Fig. 3A as the drawing is a schematic drawing showing the components separated out for clarity). First layer 40 has a bulge 42 formed by cold forming. This contains a compound 43. The opposite surface of the chamber defined by the bulge 42 is first heat sealed with a membrane 44 (e.g. of AMVC (PET/AI/PE)) provided with one or more lines of weakness 45 (formed as described in the first example) as shown in Figs. 3B.

The bulge 42 is off-set to one side of the pack and the line of weakness 50 in the barrier 44 is off-set with respect to centre of the bulge 42 such that it is reasonably central within the pack.

In order to dispense the compound 43 onto the condom 46, a user presses on the bulge 42 to open the barrier 44 at its lines of weakness 45 forcing the compound 43 onto the condom 46. The pack is then torn open in a manner of a conventional condom pack.

An advantage of this configuration is that the user can effectively use the rolled portion of the condom 46 as a support and a pivot for their thumb which can be used to apply pressure in the direction of arrow 51 which provides a more convenient dispensing operation.

## Claims

1. A pack having an outer sealed wall containing a condom (10) and a liquid or a gel (9) which is isolated from the condom (10) by a barrier (7), the barrier (7) being breachable by external manipulation of the pack to apply the liquid or gel (9) to one side of the condom (10) without disturbing the integrity of the outer sealed wall; wherein the outer sealed wall is provided by a moulded tray (1) comprising an annular trough (4), the tray being sealed with a foil lid (3), wherein a deformable portion (5) is provided by a centre portion of the tray surrounded by the annular trough (4) and wherein the deformable portion (5) projects downwardly no further than the lower surface of the trough (4), and wherein the barrier (7) is sealed across the deformable portion (5) to seal the liquid or gel (9) therein, the deformable portion (5) being deformable from a first non-deformed configuration to a second configuration in which it has breached the barrier (7) and expelled the liquid or gel (9) through the barrier (7).

2. A pack according to any preceding claim, wherein the deformable portion (5) is arranged such that, once it has deformed by more than a certain amount, it is biased to the second configuration.

3. A pack according to any one of the preceding claims, wherein the deformable portion (5) has a projection (6) to facilitate breaching of the barrier (7).

4. A pack according to any one of the preceding claims, wherein the barrier (7) is a foil or other thin and easily tearable laminate.

5. A pack according to claim 1, wherein the barrier (7) has a weakened portion (8) at its central region.

6. A pack according to claim 5, wherein the weakened portion (8) is provided by localised thinning of the barrier (7), preferably by a score line formed in said barrier (7).

7. A pack according to any preceding claims, wherein the condom (10) has a rolled wall portion (11) and said rolled wall portion (11) is accommodated within the trough (4).

8. A pack according to any one of the preceding claims, wherein the liquid or gel (9) contains an erectogenic compound or vasoactive compound.

9. A pack according to any one of the preceding claims, wherein the liquid or gel (9) is a compound including glyceryl trinitrate.

10. A pack according to any one of claims 1 to 7, wherein the liquid or gel (9) is a compound including a lubricant, spermicide and/or fragrance.

11. A pack according to any one of claims 8-10, wherein, in use, the compound (9) is primarily intended to be dispensed onto an internal surface of the condom.

## Patentansprüche

1. Packung mit einer versiegelten Außenwand, die ein Kondom (10) und eine Flüssigkeit oder ein Gel (9) enthält, die bzw. das vom Kondom (10) durch eine Barriere (7) isoliert ist, wobei die Barriere (7) durch externe Manipulation der Packung durchbrechbar ist, um die Flüssigkeit oder das Gel (9) auf eine Seite des Kondoms (10) aufzutragen, ohne die Integrität der versiegelten Außenwand zu beeinträchtigen;
wobei die versiegelte Außenwand von einer geformten Schale (1) bereitgestellt wird, die eine ringförmige Mulde (4) umfasst, wobei die Schale mit einem Foliendeckel (3) verschlossen ist, wobei ein verformbarer Abschnitt (5) von einem mittleren Abschnitt der Schale, der von der ringförmigen Mulde (4) umgeben ist, bereitgestellt wird, und wobei der verformbare Abschnitt (5) nach unten höchstens bis zu Unterseite der Mulde (4) ragt, und wobei die Barriere (7) über den verformbaren Abschnitt (5) versiegelt ist, um die Flüssigkeit oder das Gel (9) darin einzuschließen, wobei der verformbare Abschnitt (5) aus einer ersten, nicht verformten Konfiguration in eine zweite Konfiguration verformbar ist, in der er die Barriere (7) durchbrochen hat und die Flüssigkeit oder das Gel (9) durch die Barriere (7) ausgestoßen hat.

2. Packung nach einem der vorhergehenden Ansprüche, wobei der verformbare Abschnitt (5) derart angeordnet ist, dass er, nachdem er um mehr als eine bestimmte Menge verformt wurde, in die zweite Konfiguration vorgespannt wird.

3. Packung nach einem der vorhergehenden Ansprüche, wobei der verformbare Abschnitt (5) einen Vorsprung (6) zum Erleichtern des Durchbrechens der Barriere (7) aufweist.

4. Packung nach einem der vorhergehenden Ansprüche, wobei die Barriere (7) eine Folie oder ein anderes dünnes und leicht zerreißbares Laminat ist.

5. Packung nach Anspruch 1, wobei die Barriere (7) einen geschwächten Abschnitt (8) in ihrer mittleren Region aufweist.

6. Packung nach Anspruch 5, wobei der geschwächte Abschnitt (8) durch lokalisierte Ausdünnung der Barriere (7), vorzugsweise durch eine in der Barriere (7) ausgebildete Kerblinie bereitgestellt wird.

7. Packung nach einem der vorhergehenden Ansprüche, wobei das Kondom (10) einen aufgerollten Wandabschnitt (11) aufweist und der aufgerollte Wandabschnitt (11) in der Mulde (4) aufgenommen ist.

8. Packung nach einem der vorhergehenden Ansprüche, wobei die Flüssigkeit oder das Gel (9) eine erektogene Verbindung oder eine vasoaktive Verbindung enthält.

9. Packung nach einem der vorhergehenden Ansprüche, wobei die Flüssigkeit oder das Gel (9) eine Verbindung ist, die Glyceryltrinitrat enthält.

10. Packung nach einem der Ansprüche 1 bis 7, wobei die Flüssigkeit oder das Gel (9) eine Verbindung ist, die ein Gleitmittel, Spermizid und/oder einen Duftstoff enthält.

11. Packung nach einem der Ansprüche 8-10, wobei die Verbindung (9) im Gebrauch primär dazu bestimmt ist, auf eine Innenseite des Kondoms abgegeben zu werden.

## Revendications

1. Emballage ayant une paroi scellée externe contenant un préservatif (10) et un liquide ou un gel (9) qui est isolé du préservatif (10) par une barrière (7), la barrière (7) pouvant être rompue par une manipulation externe de l'emballage pour appliquer le liquide ou le gel (9) sur un côté du préservatif (10) sans perturber l'intégrité de la paroi scellée externe ;
la paroi scellée externe étant fournie par un plateau moulé (1) comprenant un creux annulaire (4), le plateau étant scellé à l'aide d'un couvercle de feuille (3), une partie déformable (5) étant fournie par une partie centrale du plateau entourée par le creux annulaire (4) et la partie déformable (5) faisant saillie vers le bas pas au-delà de la surface inférieure du creux (4), et la barrière (7) étant scellée à travers la partie déformable (5) pour sceller le liquide ou le gel (9) dans celle-ci, la partie déformable (5) étant déformable d'une première configuration non déformée à une seconde configuration dans laquelle elle a traversé la barrière (7) et expulsé le liquide ou le gel (9) à travers la barrière (7).

2. Emballage selon une quelconque revendication précédente, la partie déformable (5) étant agencée de telle sorte que, une fois qu'elle a été déformée de plus d'une certaine quantité, elle est biaisée vers la seconde configuration.

3. Emballage selon l'une quelconque des revendications précédentes, la partie déformable (5) comportant une saillie (6) pour faciliter la rupture de la barrière (7).

4. Emballage selon l'une quelconque des revendications précédentes, la barrière (7) étant une feuille ou un autre stratifié mince et facilement déchirable.

5. Emballage selon la revendication 1, la barrière (7) ayant une partie affaiblie (8) au niveau de sa région centrale.

6. Emballage selon la revendication 5, la partie affaiblie (8) étant fournie par amincissement localisé de la barrière (7), de préférence par une ligne de pliure formée dans ladite barrière (7).

7. Emballage selon l'une quelconque des revendications précédentes, le préservatif (10) ayant une partie de paroi enroulée (11) et ladite partie de paroi enroulée (11) étant logée à l'intérieur du creux (4).

8. Emballage selon l'une quelconque des revendications précédentes, le liquide ou le gel (9) contenant un composé érectogène ou un composé vasoactif.

9. Emballage selon l'une quelconque des revendications précédentes, le liquide ou le gel (9) étant un composé comprenant du trinitrate de glycéryle.

10. Emballage selon l'une quelconque des revendications 1 à 7, le liquide ou le gel (9) étant un composé comprenant un lubrifiant, un spermicide et/ou une fragrance.

11. Emballage selon l'une quelconque des revendications 8 à 10, dans lequel, lors de l'utilisation, le composé (9) est principalement destiné à être distribué sur une surface interne du préservatif.
